Europäisches Patentamt

European Patent Office

Office européen des brevets

(19)

(11) Numéro de publication: **0 341 099**
A1

# (12) DEMANDE DE BREVET EUROPEEN

(21) Numéro de dépôt: 89400893.7

(22) Date de dépôt: 31.03.89

(51) Int. Cl.⁴: **C 07 C 59/84**
C 07 C 51/10

(30) Priorité: 06.04.88 FR 8804515

(43) Date de publication de la demande:
08.11.89 Bulletin 89/45

(84) Etats contractants désignés:
AT BE CH DE ES FR GB GR IT LI LU NL SE

(71) Demandeur: RHONE-POULENC CHIMIE
25, quai Paul Doumer
F-92408 Courbevoie Cédex (FR)

(72) Inventeur: Coste, Philippe
112 Cour Tolstoi
F-69100 - Villeubanne (FR)

Baudouin, Michel
Val Fontaine 35 Rue des Gouttes
St Genis les Ollières F-69290 Chaponne (FR)

Leconte, Philippe
45, rue Duquesnes
F-69006 - Lyon (FR)

Perron, Robert
La Pecolière
F-69390 - Charly (FR)

(74) Mandataire: Le Pennec, Magali et al
Rhone-Poulenc Chimie Service Brevets Chimie 25 Quai
Paul Doumer
F-92408 Courbevoie (FR)

(54) Procédé de préparation de phénylpyruvate alcalin.

(57) La présente invention concerne un procédé industriel de préparation de phénylpyruvate alcalin par carboxylation du chlorure de benzyle en présence d'un catalyseur au cobalt.

EP 0 341 099 A1

**Description**

## PROCEDE DE PREPARATION DE PHENYLPYRUVATE ALCALIN

La présente invention concerne un nouveau procédé de préparation de phénylpyruvate alcalin par double carbonylation du chlorure de benzyle. Elle concerne plus particulièrement un procédé industriel de préparation du dit produit.

Il est connu depuis longtemps de préparer les phénylpyruvates par double carbonylation du chlorure de benzyle. Il est par exemple décrit dans le brevet US 4 152 352 un procédé de préparation de l'acide phényl pyruvique par double carbonylation du chlorure de benzyle en présence d'un catalyseur à base de cobaltcarbonyle, d'un agent basique et particulièrement d'une base alcalinoterreuse, dans un solvant hydroalcoolique.

La préparation du catalyseur qui est un sel de cobalt tétracarbonyl a été décrite en détail dans le brevet européen n° 108 698. Selon ce brevet on met en contact du dichlorure de cobalt, un dérivé soufré ou un mélange de dérivés soufrés, avec une base alcalinoterreuse, de préférence la chaux, dans un milieu constitué par de l'eau et/ou un alcool.

L'ensemble de ces deux brevets décrit la préparation du catalyseur qu'il faut malheureusement stocker, puis son utilisation dans la double carbonylation mais aucun brevet ne décrit l'ensemble de la réaction préparation du catalyseur et carbonylation du chlorure de benzyle permettant d'atteindre le phénylpyruvate de calcium dans une seule entité réactionnelle, selon un procédé de mise en oeuvre facile et avec de bons rendements.

Cette réaction simple dans son principe pose de nombreux problèmes de mise en oeuvre. En effet d'une part le milieu de réaction contient trois phases : solide, liquide et gaz ce qui exige une technologie adaptée au niveau de l'agitation et du transfert gaz-liquide, d'autre part la cinétique de la réaction doit être rapide. En outre la récupération du phénylpyruvate est extrêmement compliquée car ce produit pose de grosses difficultés de séparation de son milieu réactionnel complexe contenant des sels minéraux, des produits organiques, des solvants, de l'eau.

Le phénylpyruvate de calcium obtenu est un produit fragile qui ne peut être chauffé par crainte de le voir se dégrader et qui se présente sous une forme physique, cristaux et aiguilles de faible taille qui ne peut être ni distillé, ni extrait sous forme pure par aucun solvant. La filtration est la seule technique industrielle rentable qui puisse permettre de le séparer. Elle dépend fortement des conditions de la préparation du phénylpyruvate et donc de la qualité physique et chimique qui le caractérise à l'issue de la double carbonylation.

Les produits secondaires : solvants, sels minéraux doivent, dans la mesure où l'on désire un procédé économique et rentable, être recyclés ou éliminés en respectant toutes les normes de sécurité vis-à-vis de l'environnement.

Le phénylpyruvate de calcium obtenu devra subir une réaction de transalification pour obtenir un phénylpyruvate alcalin qui sera ensuite utilisable comme intermédiaire dans l'industrie pharmaceutique ou phytosanitaire. La principale utilisation du phénylpyruvate alcalin est la préparation de la phénylalanine. Cette synthèse est avantageusement réalisée par transamination enzymatique telle que décrite par exemple dans le brevet US 4 600 692. Toute réaction enzymatique est favorisée par le degré de pureté des matières premières ce qui entraîne des contraintes quant à la pureté du phénylpyruvate alcalin. L'ensemble de ces problèmes est résolu par le procédé de la présente invention.

La présente invention a permis d'atteindre cet objectif : obtenir une solution réactionnelle à partir de laquelle on puisse isoler le phénylpyruvate de calcium par filtration, et le transformer ensuite en phénylpyruvate alcalin.

Elle a pour objet un procédé de préparation de phénylpyruvate alcalin caractérisé en ce que :

- dans une première étape on met en contact une suspension d'hydroxyde de calcium (1) dans un mélange eau/isopropanol (2), avec du sulfure de sodium (3), du thiosulfate de sodium (4), du chlorure de cobalt (5) et de l'oxyde de carbone (6) ;
- dans une 2ème étape on introduit dans le mélange issu de la première étape le chlorure de benzyle (7) et on maintient le réacteur sous pression d'oxyde de carbone (6).
- dans une 3ème étape on oxyde le cobalt directement dans le milieu issu de la 2ème étape ;
- dans une 4ème étape on filtre le phénylpyruvate de calcium sur un filtre en couche mince, après acidification du filtrat on distille à partir de celui-ci l'azéotrope eau/isopropanol que l'on recycle à la 1ère étape ;
- dans une 5ème étape on sèche le phénylpyruvate de calcium de façon à éliminer l'isopropanol à une température inférieure ou égale à 80°C ;
- dans une 6ème étape on acidifie le phénylpyruvate de calcium mis en suspension dans un solvant organique non miscible à l'eau, on isole par décantation le phénylpyruvate acide ;
- dans une 7ème étape on neutralise le phénylpyruvate acide par une base alcaline.

Il n'était pas évident à la lecture des brevets de préparation du catalyseur de double carbonylation (EP 108 698) de préparer le catalyseur de carbonylation au sein du réacteur de carbonylation et d'ajouter directement dans le réacteur le chlorure de benzyle.

Cette découverte a permis d'éviter la préparation préalable du catalyseur dans un autre réacteur, son stockage et sa manipulation. Il ne faut pas oublier que le tétracarbonylate de cobalt calcique est un produit dangereux non volatil et que sa transformation en hydrure tétracarbonylate de cobalt fournit un produit volatil d'une toxicité extrême. L'avantage de n'utiliser qu'un seul réacteur est sur un plan économique très appréciable car cela évite de pressuriser successivement deux réacteurs avec de l'oxyde de carbone.

Les problèmes de filtration préalablement évoqués liés à la nature du phénylpyruvate de calcium ont été résolus en agissant sur plusieurs paramètres chimiques ou technologiques.

D'un point de vue chimique, pour faciliter la filtration du phénylpyruvate de calcium, il a été découvert que la concentration en cobalt dans le milieu réactionnel devait être aussi faible que possible. Une concentration de l'ordre de $10^{-3}$ mole de catalyseur au cobalt par litre de milieu réactionnel est tout à fait conseillée. On préfère utiliser une concentration variant entre $10^{-3}$ et $510^{-3}$ mole/litre de milieu réactionnel.

Cette diminution de la concentration en cobalt tétracarbonyle permet de conserver une vitesse de réaction correcte sur un plan industriel. Aucun document de l'art antérieur ne décrit une carbonylation avec une si faible quantité de catalyseur.

D'un point de vue technologique le système d'agitation a une influence sur la filtrabilité. Plus les cristaux de phénylpyruvate sont gros plus la filtrabilité sera facile. Ainsi le système d'agitation doit assurer le meilleur contact gaz-liquide mais doit détruire le moins possible de cristaux. Le système utilisé dans la présente invention est constitué d'une hélice nécessitant une faible puissance d'agitation couplée à un circulateur de gaz qui recycle le ciel gazeux du réacteur dans le milieu réactionnel.

Le solvant joue aussi un rôle important en ce qui concerne la filtrabilité. L'alcool favorise la filtrabilité et la sélectivité et l'eau favorise la vitesse de réaction. Le meilleur compromis vitesse de réaction/filtrabilité a été atteint avec un mélange eau/isopropanol contenant environ 80 volumes d'isopropanol pour 20 volumes d'eau.

Pour une meilleure mise en oeuvre de la réaction on préfère utiliser une concentration molaire en cobalt par litre de milieu réactionnel de $10^{-3}$ à $510^{-3}$ atome gramme/litre, une quantité d'hydroxyde de calcium correspondant de préférence à la stoechiométrique de la réaction, une concentration en dérivé soufré par atome gramme de cobalt comprise entre 0,01 et 1 et de préférence d'environ 0,2. On préfère travailler avec un rapport molaire du catalyseur à base de cobalt au chlorure de benzyle compris entre $10^{-3}$ et $10^{-2}$. Il est avantageux d'utiliser l'oxyde de carbone sous une pression comprise entre 5 et 30 bar. On effectue notamment la double carbonylation à une température comprise entre 60 et 90°C.

Avant de filtrer le milieu réactionnel il est préférable de procéder à une étape de détoxification pour transformer tout le cobalt à l'état d'oxydation (-1) subsistant en cobalt (+2).

Cette étape d'oxydation est réalisée de préférence en présence d'hypochlorite de sodium.

On utilise de préférence pour réaliser l'oxydation une solution aqueuse d'hypochlorite de sodium à 10% (8). Cette solution est introduite notamment dans le milieu réactionnel brut à chaud, une température d'environ 60°C est conseillée.

La suspension ainsi obtenue ne contient plus de cobalt à l'état d'oxydation - 1, présente une rhéologie non modifiée par rapport à la suspension issue de la réaction et aucune modification des composés réactionnels si ce n'est l'oxydation du cobalt.

Elle est filtrée dans une 4ème étape sous faible épaisseur par exemple sur un filtre à bandes ou sur tout dispositif permettant d'éviter un gâteau présentant une trop forte épaisseur. La filtration est réalisée de préférence à chaud à une température de l'ordre de 60°C. Le résidu de filtration est lavé avec un mélange eau/isopropanol de même nature que celui ayant servi à la double carbonylation et contenant notamment un volume d'eau pour un volume d'isopropanol (9). Le lavage est de préférence réalisée par élution pour ne pas provoquer de turbulence ni au niveau du solide ni au niveau du liquide mais déplaçant seulement les eaux mères de la solution initiale.

Le mélange azéotrope isopropanol/eau contenu dans le filtrat est récupéré par distillation après acidification du filtrat. Cette acidification est effectuée jusqu'à un PH d'environ 3. Elle est effectuée par exemple par un acide minéral choisi parmi l'acide chlorhydrique et l'acide sulfurique. Elle permet de libérer les acides, sous-produits de la réaction, de leurs sels de calcium (11). Ils se présentent alors sous forme d'une huile fluide qui ne perturbe pas la distillation de l'azéotrope, et qui en outre évite les problèmes d'encrassement de la colonne.

Le résidu de filtration est séché dans une 5ème étape de façon à éliminer le solvant restant à une température inférieure ou égale à 80°C. Il est préférable de laisser subsister une quantité d'eau résiduelle afin de ne pas dégrader le phénylpyruvate de calcium, cette quantité d'eau est avantageusement comprise entre 5 et 20 % en poids.

Le phénylpyruvate de calcium issu de la 5ème étape est acidifié de façon à libérer l'acide phénylpyruvique en solution organique et les sels en solution aqueuse, tout particulièrement les chlorures de cobalt et de calcium.

Cette réaction d'acidification est menée dans des conditions précises afin de pouvoir facilement séparer l'acide par décantation. Le phénylpyruvate de calcium est introduit dans une solution organique non miscible à l'eau avec une solution aqueuse d'acide chlorhydrique (12).

Les solvants organiques sont choisis de préférence parmi les solvants aliphatiques ou aromatiques éventuellement halogénés tels que :
- le chloroforme,
- le chlorure de méthylène,
- les chlorobenzènes,
- le toluène,
- les xylènes,
parmi les éthers tels que :
- l'éther isopropylique,
- l'éther éthylique,
- le méthyltertiobutyléther,
parmi les esters tels que :
- l'acétate d'éthyle.

On préfère utiliser le méthyltertiobutyléther.

Le rapport molaire de la quantité d'acide chlorhydrique introduite au phénylpyruvate de calcium, brut de filtration est compris entre 2 et 8, on préfère tout particulièrement utiliser un rapport molaire d'environ 4. La masse de phénylpyruvate de calcium à utiliser est de préférence comprise entre 0,1 et 1 kg.

Selon une meilleure façon de procéder il est avantageux de réaliser une émulsion méthyltertiobutyléther et acide chlorhydrique et d'introduire dans cette émulsion le phénylpyruvate de calcium. Si la quantité d'eau apportée est trop importante on note l'apparition d'un solide qui perturbe la décantation et ne permet pas de séparer l'acide phénylpyruvique. Si la quantité d'eau est trop faible l'acide phénylpyruvique se détériore. La quantité d'eau préférentielle calculée par rapport au solvant est comprise entre 1 et 3 selon un rapport pondéral.

Après acidification, l'acide phénylpyruvique en solution dans le solvant organique se sépare par décantation d'une solution aqueuse (14) contenant les sels minéraux et tout particulièrement le cobalt.

Cette solution d'acide phénylpyruvique dans le solvant organique est salifiée par une solution aqueuse alcaline (15) de préférence en continu dans un réacteur muni par exemple d'un agitateur cisaillant. La solution alcaline est de préférence de la soude. Le PH de la solution est maintenu avantageusement entre 6 et 8 et le temps de séjour est de préférence inférieur à 1 heure. Après salification le phénylpyruvate alcalin en solution aqueuse (16) est séparé du solvant organique non miscible par décantation. Il peut être directement introduit pour la réaction de transamination enzymatique.

La présente invention sera plus complètement décrite à l'aide des exemples suivants qui ne doivent pas être considérés comme limitatifs de l'invention.

## Exemple de préparation du PPCa

### 1 - Essai avec solution catalytique concentrée préparée extemporanement.

Dans un réacteur 3,6 1 inox équipé d'une hélice mixel et du recirculateur de gaz, on charge successivement :
- 222 g (3 mol) de $Ca(OH)_2$
- 1250 g d'isopropanol
- 311 g d'eau

La suspension est agitée et purgée par 3 fois 5 bar d'azote puis par 3 fois 5 bar de CO.

On introduit ensuite 29,6 g d'une solution hydroalcoolique de $[Co(CO)_4^-]_2$ Ca préparée selon le brevet EP 108 698 contenant $6\ 10^{-3}$ moles de $Co^{-1}$.

Le réacteur est porté à 70°C sous 19 bar de CO.

On introduit sous pression 250 g de chlorure de benzyl à 70° sous 19 bar de CO pendant 6 heures.

Le rapport molaire $Co^{-1}$/chlorure de benzyle est de $3\ 10^{-3}$.

En fin de réaction (absorption de CO d'environ 0) le mélange réactionnel est détendu à P = 1 bar, refroidi à 60°C le cobalt est oxydé par addition de 100 g d'une solution à 10 % de NaOCl, puis, la suspension est filtrée, le résidu est lavé avec un mélange 50/50 v/v $H_2O$/isopropanol.

On mesure pendant la filtration une résistance à l'écoulement $\alpha = 0,4$ m/kg.

Après séchage, on obtient un gâteau sec pesant 438 g et titrant 56,6 % en acide phénylpyruvique. Soit un rendement de 77,4 % par rapport au chlorure de benzyle engagé.

### 2 - Essai avec préparation du catalyseur dans le réacteur.

Dans le même appareillage que précédemment
- on charge successivement :
- $Ca(OH)_2 = 222$ g (3 mol)
- isopropanol = 1240 g
- eau = 311 g
- $CoCl_2$ (10,87 mmol)
- $Na_2S$ (0,95 mmol)
- $Na_2SO_3$ (0,87 mmol).

Le réacteur est purgé par 3 fois 5 bar d'azote puis par 3 fois 5 bar de CO.

La température est élevée à 75° sous 19 bar de CO. On laisse réagir 45 minutes. Le rendement en $Ca[Co(CO)_4]$ formé est estimé à environ 80 %. On a alors en solution environ 8 mmoles de $Co^{-1}$. Puis on introduit directement sous pression 245 g (1,937 moles) de chlorure de benzyle en 20 minutes. Le rapport molaire$Co^{-1}$/chlorure de benzyle est de $4\ 10^{-3}$. Au bout de 5 heures on obtient une sélectivité en acide phénylpyruvique de 75 %. Le mélange réactionnel est traité de la même façon que précédemment.

### 3 - Traitement du filtrat

1 kg de jus de filtration de composition :
- Isopropanol = 69 % p/p
- $H_2O$ = 22 %
- $CoCl_2$ = 5 %
- Organiques = 3,6 %

sont amenés à PH = 3 par addition de 22,6 g de Hcl 38 %. Les jus acidifiés sont évaporés en continu dans un évaporateur monoétage (P = Patm, Tvapeurs = 80°C). Le taux de vaporisation est de 78 %.

Le distillat est composé de l'azéotrope izopropanol/eau (85/15 p/p). Le taux de récupération de l'izopropanol est de 99 %.

Le pied de distillation décante en deux phases :
- phase organique : 47 g
- phase aqueuse : 174 g

La phase organique est brûlée, la phase aqueuse (saturée en $CoCl_2$) est rejettable.

### 4 - Passage du phénylpyruvate de calcium (PPCa) au phénylpyruvate de sodium (PPNa)

### 1 - Acidification du PPCa

On ajoute 0,167 mol de phénylpyruvate de calcium séché obtenu dans les conditions précédemment décrites à une suspension méthyltertiobutyléther/ $HCl$/$H_2O$ (300 ml/0,67 mol/355 g) dans un réacteur double enveloppe agité par hélice mixel.

La température est maintenue à 20° sous balayage d'azote pendant 1 heure. La phase aqueuse contenant le $CaCl_2$ le $CoCl_2$ est décantée. On obtient 238,2 g de phase organique légèrement jaune contenant l'acide phénylpyruvique (APP) (RR $\frac{APP}{PPCa} = 98$ %)

### 2 - Salification de l'APP en PPNa

La phase organique d'acide phénylpyruvique est introduite en continu (200 cm³/h) dans un réacteur de 500 ml agité par une hélice Rushton. Parallèlement, on ajoute en continu dans ce réacteur une solution de soude 1,086 (P/P) de façon à maintenir le PH à 7,5 (20 minutes, balayage d'azote).

Le rendement $\underline{PPNa}$

APP

sur des coupes pendant 4 heures de réaction est de 95 %. La couche aqueuse finale contient 5 % de PPNa dans l'eau. La couche organique de méthyltertiobutyléther est directement recyclable à l'acidification.

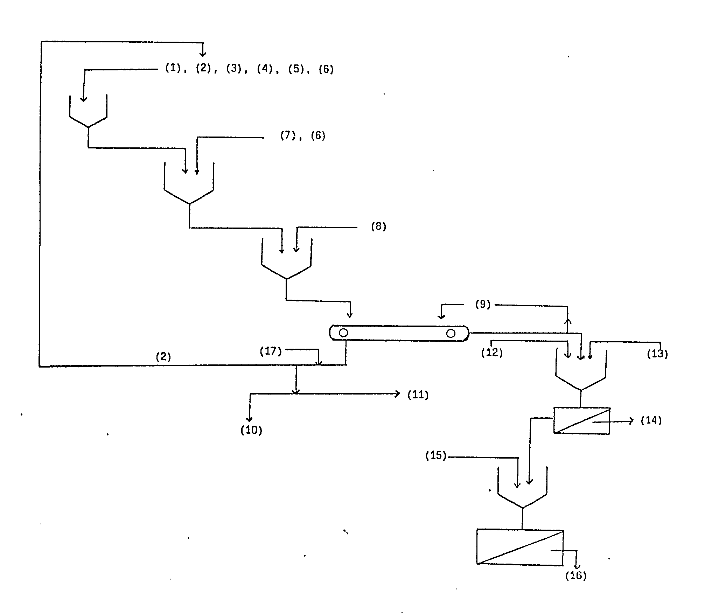

## Revendications

1 - Procédé de préparation de phénylpyruvate alcalin caractérisé en ce que :
- dans une première étape on met en contact une suspension d'hydroxyde de calcium dans un mélange eau/isopropanol, avec du sulfure de sodium, du thiosulfate de sodium, du chlorure de colbalt et de l'oxyde de carbone ;
- dans une 2ème étape on introduit dans le mélange issu de la 1ère étape le chlorure de benzyle et on maintient le réacteur sous pression d'oxyde de carbone ;
- dans une 3ème étape on oxyde le cobalt directement dans le milieu issu de la 1ère étape ;
- dans une 4ème étape on filtre le phénylpyruvate de calcium sur un filtre couche mince, après acidification du filtrat on distille l'azéotrope eau/isopropanol que l'on recycle à la 1ère étape et on rejette les sels de cobalt et de calcium en solution aqueuse ;
- dans une 5ème étape on sèche le phénylpyruvate de calcium de façon à éliminer l'isopropanol à une température inférieure ou égale à 80°C ;
- dans une 6ème étape on acidifie le phénylpyruvate de calcium mis en suspension dans un solvant organique non miscible à l'eau, on isole par décantation le phénylpyruvate acide ;
- dans une 7ème étape on neutralise le phénylpyruvate acide par une base alcaline.

2 - Procédé selon la revendication 1 caractérisé en ce qu'on utilise une concentration de cobalt comprise entre $10^{-3}$ et $5\,10^{-3}$ mole/litre.

3 - Procédé selon la revendication 1 caractérisé en ce que la quantité de dérivé soufré exprimée en mole par atome gramme de cobalt est comprise entre 0,01 et 1.

4 - Procédé selon la revendication 1 caractérisé en ce que la quantité molaire de catalyseur à base de cobalt mise en oeuvre calculée par mole de chlorure de benzyle est comprise entre $10^{-3}$ et $10^{-2}$.

5 - Procédé selon la revendication 1 caractérisé en ce que la pression d'oxyde de carbone utilisée est comprise entre 5 et 30 bar.

6 - Procédé selon la revendication 1 caractérisé en ce que la température de la réaction de carbonylation est comprise entre 60 et 90°C.

7 - Procédé selon la revendication 1 caractérisé en ce que l'oxydation de la 3ème étape est effectuée par addition d'hypochlorite de sodium.

8 - Procédé selon la revendication 7 caractérisé en ce que l'addition de l'hypochlorite de sodium est effectuée à une température d'environ 60°C.

9 - Procédé selon la revendication 1 caractérisé en ce que la filtration de la 4ème étape est effectuée sur une filtre en couche mince.

10 - Procédé selon la revendication 1 caractérisé en ce que l'acidification du filtrat de la 4ème étape est effectuée jusqu'à PH = 3

11 - Procédé selon la revendication 1 caractérisé en ce que lors de la 6ème étape on utilise un solvant choisi parmi les hydrocarbures aliphatiques ou aromatiques éventuellement halogénés, les éthers, les esters.

12 - Procédé selon la revendication 1 caractérisé en ce que le solvant choisi est le méthyltertiobutyléther.

13 - Procédé selon la revendication 1 caractérisé en ce que lors de la 6ème étape l'acidification est effectuée par une solution d'acide chlorhydrique et selon un rapport molaire de l'acide chlorhydrique au phénylpyruvate comprise entre 2 et 8 et de préférence d'environ 4.

(1), (2), (3), (4), (5), (6)

(7), (6)

(8)

(9)

(2)

(17)

(12)

(13)

(11)

(10)

(14)

(15)

(16)

EP 0 341 099 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | EP-A-0 108 698 (RHONE-POULENC) <br> * pages 14,15, exemple 2 * <br> --- | 1,5,6 | C 07 C 59/84 <br> C 07 C 51/10 |
| D,Y | US-A-4 152 352 (PERRON) <br> * colonne 3, lignes 54,55; exemple 1 * <br> --- | 1,5,6 | |
| A | US-A-4 351 952 (FOA) <br> * colonne 3, lignes 12-19 * <br> --- | 1 | |
| A | EP-A-0 195 530 (JAPAN AS REPRESENTED BY THE DIRECTOR GENERAL, AGENCY OF INDUSTRIAL SCIENCE AND TECHNOLOY) <br> * revendications * <br> --- | 1 | |
| A | PATENT ABSTRACTS OF JAPAN <br> tome 9, no. 192 (C-296)(1915), 8 août 1985; & JP - A - 60 61550 (SAGAMI CHUO) 09.04.1985 <br> --- | 1 | |
| A | DD-A- 144 536 (DYNAMIT NOBEL) <br> * revendications * <br> ----- | 1 | DOMAINES TECHNIQUES RECHERCHES (Int. Cl.4) <br><br> C 07 C 59/00 <br> C 07 C 51/00 <br> C 07 C 65/00 |

**Le présent rapport a été établi pour toutes les revendications**

| Lieu de la recherche | Date d'achèvement de la recherche | Examinateur |
|---|---|---|
| BERLIN | 16-06-1989 | PROBERT C.L. |

EPO FORM 1503 03.82 (P0402)